# EUROPÄISCHE PATENTSCHRIFT

(11) **EP 1 115 431 B1**
(45) Veröffentlichungstag und Bekanntmachung des Hinweises auf die Patenterteilung: **18.02.2004**
(21) Anmeldenummer: 99945986.0
(22) Anmeldetag: 17.08.1999
(51) Int. Cl.: A61L 12/12

(54) **VORRICHTUNG ZUR PFLEGE VON KONTAKTLINSEN**
DEVICE FOR CONTACT LENS CARE
DISPOSITIF POUR L'ENTRETIEN DE LENTILLES DE CONTACT

(30) Priorität: 21.09.1998 DE 19843140
(43) Veröffentlichungstag der Anmeldung: 18.07.2001
(73) Patentinhaber: Coronis GmbH, 81243 München (DE)
(72) Erfinder: MÜLLER-LIERHEIM, Wolfgang, G., K., D-81477 München (DE)
(74) Vertreter: Nöth, Heinz, Dipl.-Phys.
(86) Internationale Anmeldenummer: PCT/EP1999/006019
(87) Internationale Veröffentlichungsnummer: WO 2000/016815

(56) Entgegenhaltungen:
- WO-A-93/06870
- DATABASE WPI Section Ch, Week 199210 Derwent Publications Ltd., London, GB; Class A92, AN 1992-076947 XP002124898 & JP 04 022361 A (ISHIZUKA GLASS KK), 27. Januar 1992 (1992-01-27)

## Beschreibung

### [Stand der Technik]

Die Erfindung betrifft eine Vorrichtung zur Pflege von Kontaktlinsen nach dem Oberbegriff des Patentanspruches 1.

Bei einer derartigen aus der DE 196 24 095 C1 bekannten Vorrichtung ist ein Behälter vorgesehen, in welchem für die Kontaktlinsenpflege eine 3%ige H₂O₂-Lösung zur Desinfektion von Kontaktlinsen vorgesehen ist. In diese Lösung wird die Kontaktlinse zur Behandlung und Aufbewahrung eingetaucht. Zur Neutralisation der wäßrigen Wasserstoffperoxydlösung befindet sich auf einem Formkörper aus Glas eine Platinschicht, welche auch als Innenbeschichtung des aus Glas bestehenden Behälters vorgesehen sein kann.

Neben der Desinfektionswirkung sind bei der Kontaktlinsenpflege jedoch auch die antimikrobielle und antivirale Wirksamkeit und die ausreichende Konservierung von Bedeutung, insbesondere dann, wenn die Kontaktlinse längere Zeit in der Pflegelösung aufbewahrt wird, wie das beispielsweise bei Anpaßkontaktlinsen (DE 196 01 568 A1) der Fall ist.

Die oligodynamische Wirkung, d.h. die wachstumshemmende oder abtötende Wirkung von Schwermetallen, insbesondere von Silber, auf Mikroorganismen ist bekannt. Es zeigte sich jedoch, daß beim Einsatz von Silber (Silberung) des eingangs genannten Pflegemittelsystems in herkömmlichem Sinne die Lebensdauer des Platin-Katalysators beeinträchtigt wurde und die Platinschicht-Katalysatoren nach kurzer Zeit ersetzt werden mußten.

### [Aufgabe der Erfindung]

Aufgabe der Erfindung ist es daher, eine Vorrichtung der eingangs genannten Art zu schaffen, bei welcher neben Oligodynamie des Pflegesystems eine lange Lebensdauer des Platinschicht-Katalysators gewährleistet wird.

Diese Aufgabe wird erfindungsgemäß durch die kennzeichnenden Merkmale des Patentanspruches 1 gelöst.

Die Erfindung beruht auf der Erkenntnis, daß die Lebensdauer des Platinschicht-Katalysators nicht negativ beeinträchtigt wird, wenn die gesamte Silberoberfläche, welche in der Wasserstoffperoxydlösung wirksam wird, eine obere Grenze nicht überschreitet. Diese Obergrenze in der Größenordnung von bis zu 30 mm²/ml der in den Behälter eingefüllten H₂O₂-Lösung. Die wäßrige H₂O₂-Lösung ist in bevorzugter Weise eine physiologische Lösung mit physiologischem pH-Wert (etwa 6 bis 8). Auch in dieser Lösung wird durch die bevorzugt als einheitliche und auf einem Träger vorhandene Silbermetallschicht die gewünschte antimikrobielle Langzeit-Wirksamkeit erreicht. Innerhalb der Desinfektionsphase (2 bis 6 Stunden) wurde nach Beimpfung mit 10⁵ bis 10⁶ KBE/ml der im internationalen Normenentwurf ISO/CD 14729 vorgesehenen Testkeime (Pseudomonas aeruginosa, Staphylococcus aureaus, Serratia marcescens, Candida albicans und Fusarium solani) und ferner Aspergillus niger am Ende der Desinfektionsphase kein Wachstum gefunden, bzw. es wurde eine Keimreduktion um mindestens 5 Zehnerpotenzen (5 log) festgestellt. Nach Beimpfung mit Poliovirus ergab sich innerhalb von 60 Minuten eine Reduktion bis unter der Nachweisgrenze. Nach Beimpfung mit Adenovirus ergab sich innerhalb von 120 Minuten eine > 3 log-Reduktion, nach 240 Minuten eine >4,5 log-Reduktion (Virus unter der Nachweisgrenze). Nach Beimpfung mit HSV 1-Virus ergab sich innerhalb von 30 Minuten eine > 3 log-Reduktion (Virus unter der Nachweisgrenze).

Hierdurch wird ein einstufiges System geschaffen zur Desinfektion und Aufbewahrung von Kontaktlinsen, das bei der Desinfektion sehr wirksam ist und bei der Aufbewahrung der Kontaklinsen deren Verkeimung ohne Verwendung von Konservierungsmitteln gewährleistet.

In bevorzugter Weise wird eine Silberoberfläche von etwa 0,2 bis 0,6, insbesondere 0,3 mm²/ml der in den Behälter eingefüllten wäßrigen H₂O₂-Lösung (3 %) verwendet.

Die Silberschicht mit der angegebenen Oberfläche kann an einem Kontaktlinsenniederhalter, mit welchem die Kontaktlinse in die Lösung eingetaucht wird, vorgesehen sein. Dieser Niederhalter kann in bekannter Weise an der Unterseite eines Verschlußdeckels, mit dem der Behälter abgedeckt ist, vorgesehen sein. Durch den Niederhalter wird verhindert, daß durch die während der Kontaktlinsenpflege und der Neutralisation der Pflegelösung entstehenden Gase die zu behandelnde Kontaktlinse an die Oberfläche der Pflegelösung getrieben wird, sondern ständig eingetaucht in der Pflegelösung verbleibt.

Die Vorrichtung zur Pflege der Kontaktlinsen kann in der Weise ausgebildet sein, wie sie in der DE 197 57 356.8 A1 beschrieben ist. Hierbei kann der Behälter, welcher die wäßrige H₂O₂-Lösung und die Kontaktlinse aufnimmt, aus Glas bestehen und an seiner Innenseite mit einem Platinschicht-Katalysator beschichtet sein. Die Platinschicht kann durch Sputtern aufgebracht sein. Es ist jedoch auch möglich, einen Formkörper aus Glas, welcher mit der Platinschicht versehen ist, im Behälter vorzusehen (DE 196 24 095 C1). Die Silberschicht befindet sich dann bevorzugt am Kontaktlinsenniederhalter, welcher mit Bohrungen versehen ist, so daß die Pflegelösung auch über der Niederhaltefläche, welche in die Lösung eingetaucht ist, vorhanden ist.

Bei einer weiteren Ausführungsform kann der Kontaktlinsenniederhalter aus Glas bestehen, wobei an der einen Seite des Niederhalters, insbesondere an der Unterseite, die bevorzugt durch Sputtern aufgebrachte Platinschicht und an der anderen Seite, insbesondere an der Oberseite des Niederhalters, die Silberschicht, welche ebenfalls durch Sputtern aufgebracht sein kann, angeordnet sind. Der Behälter kann dann auch aus Kunststoff bestehen.

### [Beispiele]

Anhand der Figuren wird die Erfindung noch an Ausführungsbeispielen erläutert. Es zeigt:
- Fig. 1:: ein erstes Ausführungsbeispiel; und
- Fig. 2:: ein zweites Ausführungsbeispiel

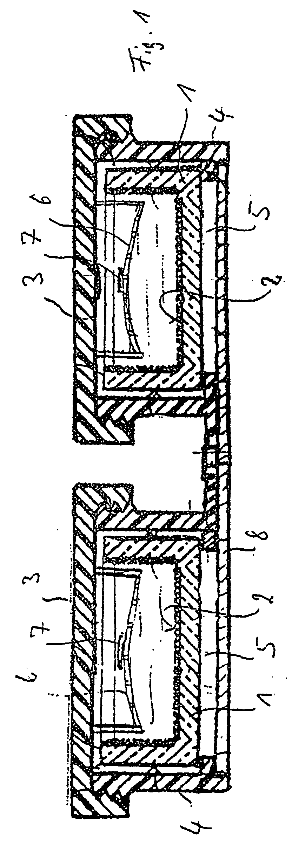

Bei den beiden Ausführungsbeispielen der Figuren 1 und 2 sind in einem Gehäuse 4 in zwei Aufnahmefächern 5 des Gehäuses zwei napfförmige Behälter 1, die flüssigkeitsundurchlässig sind, angeordnet. Jeder Behälter dient zur Aufnahme einer zu pflegenden Kontaktlinse. An der Außenseite jedes Aufnahmefaches 5 ist am oberen Rand des Gehäuses 4 ein Gewinde vorgesehen, auf welches ein Deckel 3 aufgeschraubt werden kann. Es ist jedoch auch möglich, eine andere Halteeinrichtung für den Deckel am Gehäuse vorzusehen, beispielsweise eine Aufklipsvorrichtung, Halteklammern und dergl. Hierdurch wird eine flüssigkeitsdichte Verbindung zwischen dem Deckel und dem Gehäuse hergestellt. Innerhalb der Aufnahmefächer 5 werden die jeweiligen Behälter 1 mit Abstand von der Deckelunterseite und von der Innenseite des jeweiligen Aufnahmefaches 5 gehalten. Hierdurch entstehen zwischen dem jeweiligen oberen Rand 6 des Behälters und zwischen der jeweiligen Behälteraußenseite und der Innenseite des jeweiligen Aufnahmefaches 5 ein Zwischenraum bzw. Zwischenräume, die einen Durchlaß für Gase bilden, die während der Kentaktlinsenpflege in den jeweiligen Behältern entstehen. Am jeweiligen Gehäuse 4 ist an der Unterseite ein Boden 8 vorgesehen. Das Gehäuse besteht bevorzugt aus Kunststoff. Der Boden kann aus Kunststoff, insbesondere metallisiertem Kunststoff, oder aus Metall bestehen. Der Boden kann, wie in der DE 197 57 356.8 beschrieben, herausnehmbar, beispielsweise mit Hilfe einer Schnappverbindung, am Gehäuse 4 befestigt sein. Hierdurch wird gewährleistet, daß Gas, welches während der Kontaktlinsenpflege und während der Aufbewahrung im Behälter entsteht, durch die geschaffenen Zwischenräume entweichen kann.

Bei den dargestellten Ausführungsbeispielen ist jeweils ein Niederhalter 6 vorgesehen. Der Niederhalter 6 besitzt eine Niederhaltefläche, welche in die eingefüllte Pflegelösung eingetaucht ist. Die Pflegelösung besteht aus einer 3%-igen H₂O₂-Lösung. Durch die Wirkung dieser Pflegelösung werden die eingetauchten Kontaktlinsen desinfiziert. Um Wasserstoffperoxydreste an den Kontaktlinsen nach der Pflege zu vermeiden, wird durch die Wirkung einer Platinschicht 2 das Wasserstoffperoxyd in Sauerstoff und Wasserstoff zersetzt (neutralisiert).

Beim Ausführungsbeispiel der Fig. 1 befindet sich die Platinschicht 2 an der Innenseite des aus Glas bestehenden Behälters 1. Am Niederhalter 6 befindet sich eine Silberschicht 7, welche ähnlich wie die Platinschicht 2 durch Sputtern auf die Oberseite des Niederhalters 6 aufgebracht sein kann. Der Niederhalter 6 besitzt Bohrungen, so daß die Pflegelösung durch die Niederhaltefläche hindurchdringen kann und gewährleistet ist, daß die von der Niederhaltefläche eingetauchte Kontaktlinse unterhalb der Oberfläche der eingefüllten Pflegelösung sich befindet. Auch die aufgebrachte Silberschicht 7 befindet sich immer innerhalb des Füllvolumens der eingefüllten Pflegelösung. Hierdurch wird sowohl während der Desinfektionsphase, bei welcher das Wasserstoffperoxyd wirkt, als auch nach der Neutralisierung der Wasserstoffperoxydlösung eine oligodynamische Wirkung der Lösung erzielt.

Bei der Ausführungsform der Fig. 2 befindet sich die Platinschicht 2, welche als Neutralisationskatalysator wirkt, auf der Unterseite des Niederhalters 6. Die Silberschicht 7 befindet sich an der Oberseite des Niederhalters 6. Bei diesem Ausführungsbeispiel der Fig. 2 ist der Niederhalter 6 aus Glas gebildet. Die Silberschicht 7 ist in bevorzugter Weise durch Sputtern hergestellt. Die Oberfläche der Silberschicht 7, welche mit der H₂O₂-Lösung in Berührung steht, beträgt höchstens 30 mm²/ml, bezogen auf das Füllvolumen der H₂O₂Lösung. Der Behälter 1 kann bei der Ausführungsform der Fig. 2 aus Glas oder auch aus einem anderen Material, beispielsweise Kunststoff, bestehen.

Anstelle des Niederhalters 6 können auch Körbchen verwendet werden, die jeweils an der Unterseite des Deckels 3 vorgesehen sind und die Silberschicht 7 tragen. Durch den Niederhalter bzw. das Aufbewahrungskörbchen wird gewährleistet, daß die Kontaktlinse immer in die Pflegelösung eingetaucht wird. Bei der Neutralisation und der Pflege entstehende Gase können die Linse nicht an die Oberfläche der Pflegelösung treiben. Hierdurch wird eine stetige Pflege der eingetauchten Kontaktlinse erreicht.

In den folgenden Tabelle 1 und 2 wird die oligodynamische Wirkung verschiedener Ausführungsformen des Pflegesystems bei verschiedenen Testorganismen veranschaulicht. Hierbei werden neutralisierte Wasserstoffperoxid-Lösungen (isotonisch, pH 6,5, < 5 ppm Restgehalt an Wasserstoffperoxid) mit etwa 10⁶ Mikroorganismen pro ml Lösung angeimpft. Aus den Tabellen ergibt sich auch bei relativ geringen Silberschichtoberflächen (zwischen 0,15 und 2,0 mm²/ml) die über einen längeren Zeitraum erzielte oligodynamische Wirkung, welche insbesondere für die Pflege von Anpaßkontaktlinsen von Bedeutung ist.

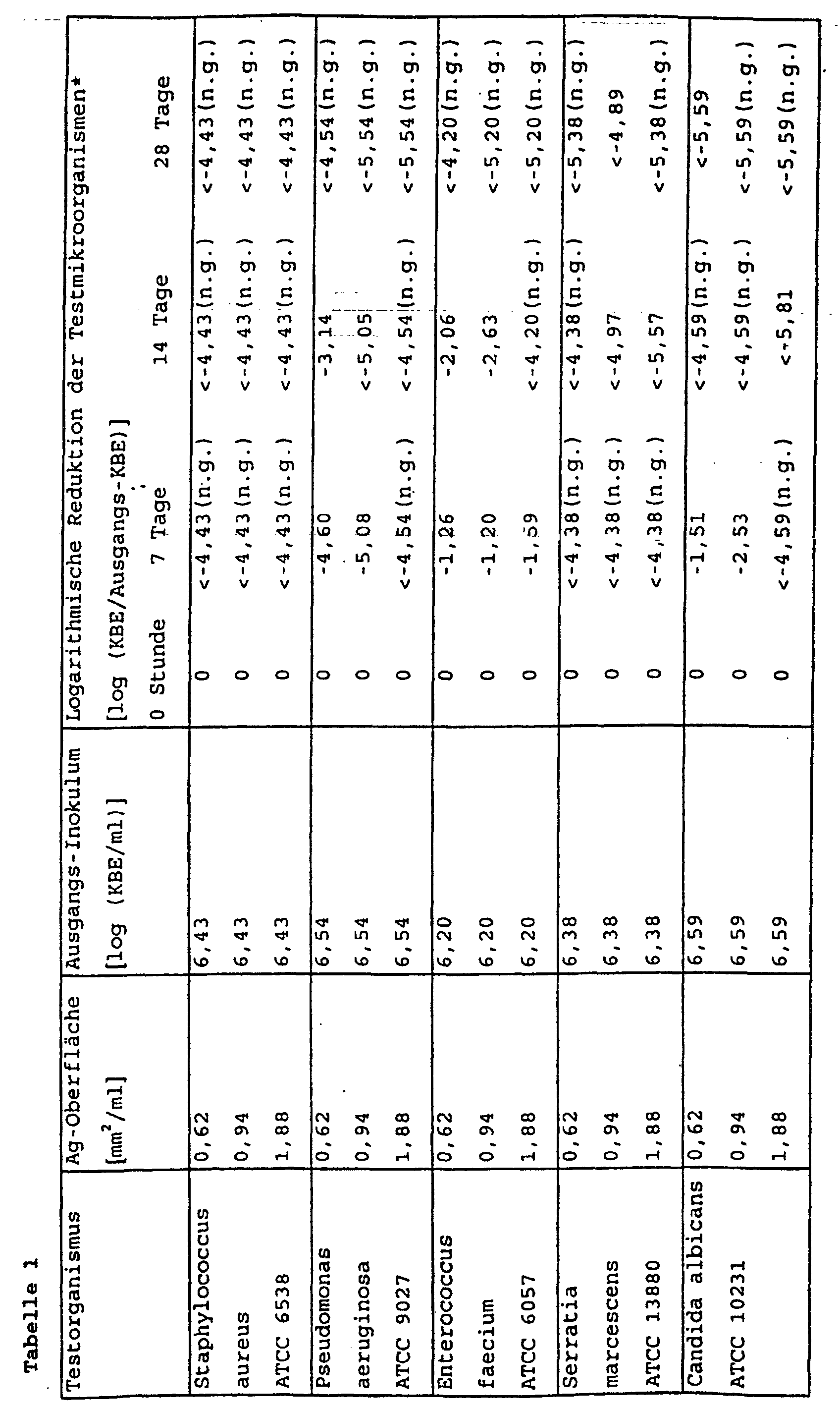

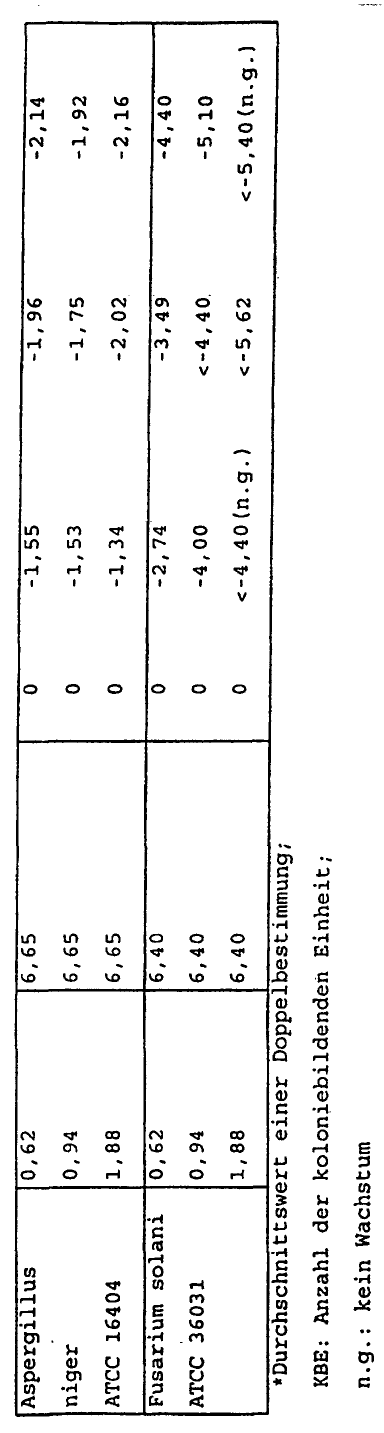

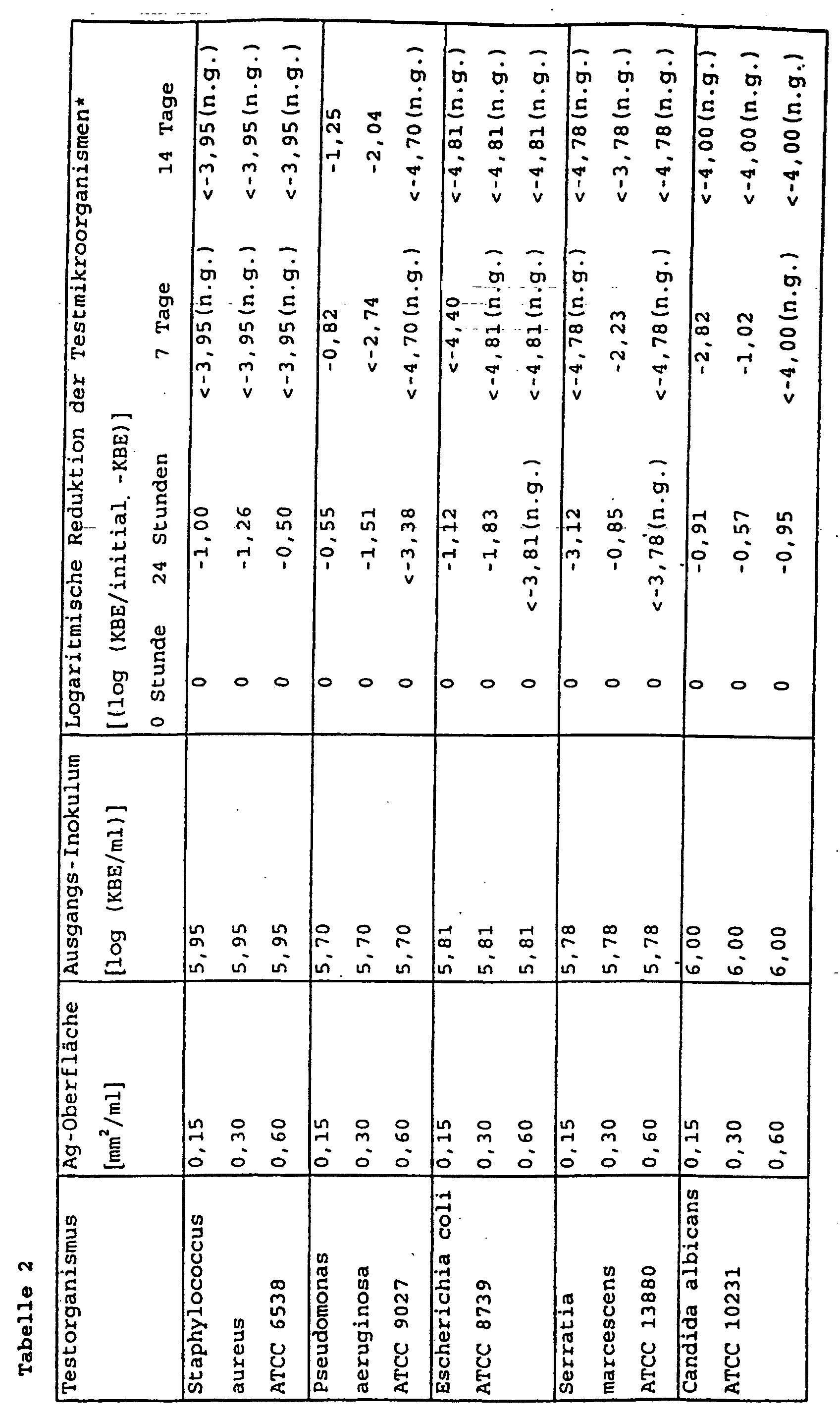

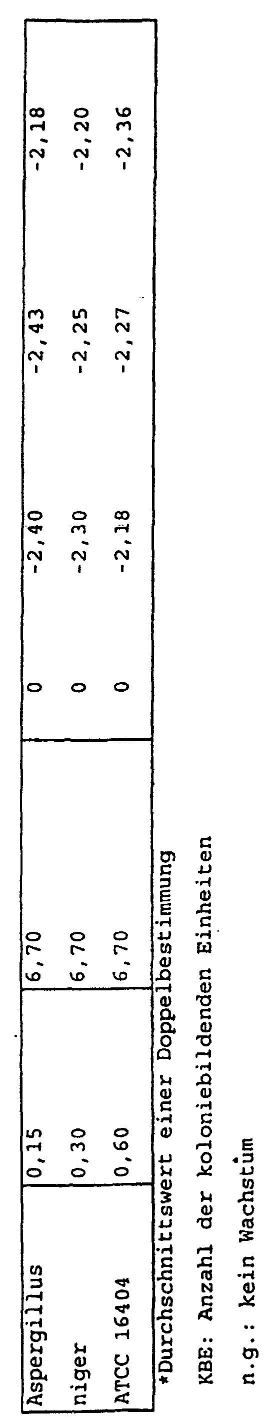

### [Bezugszeichenliste]

- 1: Behälter
- 2: Platinschicht
- 3: Deckel
- 4: Gehäuse
- 5: Aufnahmefächer
- 6: Niederhalter
- 7: Silberschicht
- 8: Boden

## Patentansprüche

1. Vorrichtung zur Pflege von Kontaktlinsen mit einem Behälter, einer in den Behälter einfüllbaren bzw. eingefüllten wässrigen H₂O₂-Lösung und einem aus einer Platinschicht bestehenden Katalysator, der mit seiner Oberfläche mit der wässrigen H₂O₂-Lösung in Berührung steht, **dadurch gekennzeichnet, daß** mit der im Behälter (1) befindlichen wässrigen H₂O₂-Lösung ferner eine auf einem Träger aufgebrachte Silberschicht (7) mit einer Oberfläche in direkter Berührung liegt, die weniger als etwa 30 mm²/ml der eingefüllten H₂O₂-Lösung beträgt.

2. Vorrichtung nach Anspruch 1, **dadurch gekennzeichnet, daß** die Silberschicht (7) an einem Niederhalter (6), mit welchem die Kontaktlinse in die H₂O₂-Lösung eingetaucht wird, vorgesehen ist.

3. Vorrichtung nach Anspruch 2, **dadurch gekennzeichnet, daß** der Niederhalter (6) an der Unterseite eines Verschlußdeckels (3) vorgesehen ist.

4. Vorrichtung nach einem der Ansprüche 1 bis 3, **dadurch gekennzeichnet, daß** die Silberschicht an einer Seite des Niederhalters (6) und die Platinschicht (2) an der anderen Seite des Niederhalters (6) vorgesehen sind und daß der Niederhalter (6) aus Glas besteht.

5. Vorrichtung nach einem der Ansprüche 1 bis 4, **dadurch gekennzeichnet, daß** die wässrige 3%-ige H₂O₂-Lösung eine physiologische Lösung ist.

6. Vorrichtung nach einem der Ansprüche 1 bis 5, **dadurch gekennzeichnet, daß** die 3%-ige H₂O₂-Lösung einen pH-Wert von etwa 6 bis 8 aufweist.

## Claims

1. A device for the care of contact lenses comprising a container, an aqueous H₂O₂ solution which is or can be introduced into the container, and a catalyst comprising a platinum layer which is in contact with its surface with the aqueous H₂O₂ solution, **characterised in that** a silver layer (7) applied on a carrier is also in direct contact with the aqueous H₂O₂ solution in the container (1), with a surface area which is less than about 30 mm²/ml of the introduced H₂O₂ solution.

2. A device according to claim 1 **characterised in that** the silver layer (7) is provided on a holding-down means (6) with which the contact lens is immersed in the H₂O₂ solution.

3. A device according to claim 2 **characterised in that** the holding-down means (6) is provided at the underside of a closure lid (3).

4. A device according to one of claims 1 to 3 **characterised in that** the silver layer is provided at one side of the holding-down means (6) and the platinum layer (2) at the other side of the holding-down means (6) and that the holding-down means (6) comprises glass.

5. A device according to one of claims 1 to 4 **characterised in that** the aqueous 3% H₂O₂ solution is a physiological solution.

6. A device according to one of claims 1 to 5 **characterised in that** the 3% H₂O₂ solution is of a pH-value of from about 6 to 8.

## Revendications

1. Dispositif pour l'entretien de lentilles de contact comprenant un récipient, une solution H₂O₂ aqueuse dont le récipient peut être rempli ou est rempli, et un catalyseur composé d'une couche de platine qui est au contact par sa surface de la solution H₂O₂ aqueuse, **caractérisé en ce qu'**une couche d'argent (7) appliquée sur un support est en outre au contact direct de la solution H₂O₂ aqueuse contenue dans le récipient (1) par une surface qui représente moins d'environ 30 mm²/ml de la solution H₂O₂ remplie.

2. Dispositif suivant la revendication 1, **caractérisé en ce que** la couche d'argent (7) est prévue sur un élément de serrage (6), avec lequel la lentille de contact est immergée dans la solution H₂O₂.

3. Dispositif suivant la revendication 2, **caractérisé en ce que** l'élément de serrage (6) est prévu sur le côté inférieur d'un bouchon (3).

4. Dispositif suivant l'une des revendications 1 à 3, **caractérisé en ce que** la couche d'argent est prévue sur un côté de l'élément de serrage (6) et la couche de platine (2) sur l'autre côté de l'élément de serrage (6), et **en ce que** l'élément de serrage (6) se compose de verre.

5. Dispositif suivant l'une des revendications 1 à 4, **caractérisé en ce que** la solution H₂O₂ aqueuse à 3% est une solution physiologique.

6. Dispositif suivant l'une des revendications 1 à 5, **caractérisé en ce que** la solution H₂O₂ à 3% a une valeur de pH d'environ 6 à 8.
